(19) Europäisches Patentamt / European Patent Office / Office européen des brevets

(11) **EP 4 748 822 A2**

(12) **EUROPEAN PATENT APPLICATION**

(43) Date of publication:
27.05.2026 Bulletin 2026/22

(21) Application number: 25216323.3

(22) Date of filing: 17.11.2025

(51) International Patent Classification (IPC):
C07C 51/43 (2006.01)          C07C 51/44 (2006.01)
C07C 51/47 (2006.01)          C07C 51/487 (2006.01)
C07C 55/10 (2006.01)          C08G 63/16 (2006.01)

(52) Cooperative Patent Classification (CPC):
(C-Sets available)
C07C 55/10; C07C 51/43; C07C 51/44;
C07C 51/47; C07C 51/487; C08G 63/16          (Cont.)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH LA MA MD TN

(30) Priority: 25.11.2024 CN 202411693376

(71) Applicants:
• Zhuhai Kingfa Biomaterial Co., Ltd.
Zhuhai, Guangdong 519050 (CN)
• KINGFA SCI. & TECH. CO., LTD.
Guangzhou, Guangdong 510663 (CN)
• Liaoning Kingfa Biomaterial Co., Ltd.
Panjin City, Liaoning Province 124000 (CN)

(72) Inventors:
• Lu, Changli
Zhuhai,Guangdong, 519050 (CN)

• Chen, Pingxu
Zhuhai,Guangdong, 519050 (CN)
• Ye, Nanbiao
Zhuhai,Guangdong, 519050 (CN)
• Zeng, Xiangbin
Zhuhai,Guangdong, 519050 (CN)
• Fu, Xuejun
Zhuhai,Guangdong, 519050 (CN)
• Zhang, Jialong
Zhuhai,Guangdong, 519050 (CN)
• Li, Zhen
Zhuhai,Guangdong, 519050 (CN)
• Zhang, Yi
Zhuhai,Guangdong, 519050 (CN)
• Yao, Yi
Zhuhai,Guangdong, 519050 (CN)

(74) Representative: Petraz, Gilberto Luigi et al
GLP S.r.l.
Viale Europa Unita, 171
33100 Udine (IT)

(54) **BIO-BASED SUCCINIC ACID COMPOSITION, PREPARATION METHOD THEREFOR, AND USE THEREOF**

(57)    The present invention provides a bio-based succinic acid composition, a preparation method therefor, and use thereof. The bio-based succinic acid composition includes bio-based succinic acid and a readily oxidizable substance; and a content of the readily oxidizable substance in the bio-based succinic acid composition is ≤ 0.32 mL/g. In the present invention, the bio-based succinic acid composition has a long shelf life, a small change in b value after aging, and good aging stability. Moreover, a polyester prepared from the bio-based succinic acid composition has a lower acid number, a whiter color, and a higher molecular weight.

(52) Cooperative Patent Classification (CPC): (Cont.)

C-Sets
**C07C 51/43, C07C 55/10;**
**C07C 51/44, C07C 55/10;**
**C07C 51/47, C07C 55/10;**
**C07C 51/487, C07C 55/10**

**Description**

## TECHNICAL FIELD

The present invention belongs to the technical field of preparation of polyesters, and particularly relates to a bio-based succinic acid composition, a preparation method therefor, and use thereof.

## BACKGROUND

Succinic acid is an important $C_4$ compound, and serving as a raw material for organic synthesis, an intermediate product, or a special chemical, has been widely used in fields, such as food, light industry, and pharmaceutical industry. The succinic acid is a raw material for preparing basic chemical products, such as 1,4-butanediol, tetrahydrofuran, γ-butyrolactone, and N-methylpyrrolidone, and is also a main raw material for completely biodegradable plastics, such as poly(butylene succinate) (PBS). Currently, raw materials for the succinic acid are mostly derived from petroleum resources (for example, using maleic anhydride as a raw material), and a chemical method is adopted for production. However, the petroleum resources are non-renewable and will generate significant pollution during production. Therefore, in order to avoid the depletion of fossil fuel resources and mitigate the increase of carbon dioxide as well as avoid environmental problems, such as the greenhouse effect, caused by the increased carbon dioxide, preparing bio-based succinic acid from biomass resources as raw materials has become a feasible strategy. Moreover, methods for preparing the succinic acid from the biomass resources as the raw materials can utilize the renewable biomass materials, have high efficiency, independence from petroleum, low pollution, and other advantages, and can absorb and fix greenhouse gases (including $CO_2$ gas) during biosynthesis, thus becoming a research and application hotspot both at home and abroad in recent years.

The bio-based succinic acid is usually prepared by a fermentation method and/or a chemical conversion method using the biomass resources as the raw materials. Accordingly, the bio-based succinic acid usually contains various impurities, such as cell debris, proteins, and amino acids directly derived from a fermentation solution, and nitrogen elements or ammonia and metal cations derived from fermentation bacteria, etc. The presence of impurities will reduce the quality of succinic acid. Additionally, these impurities, especially readily oxidizable substances, have adverse impacts on polyesters prepared from the succinic acid derived from the biomass resources. For example, when the readily oxidizable substances are present in large quantities, solid by-products are easily deposited in a reactor during the synthesis of the polyesters, and the production rates of polyesters are limited, so that polyesters with high molecular weights cannot be obtained. Alternatively, in order to obtain the polyesters with high molecular weights, a longer residence time is usually required, leading to higher acid numbers and poor hydrolysis resistance of the polyesters. In addition, in a case of a batch polymerization process, due to the presence of impurities, such as the readily oxidizable substances, process conditions need to be substantially changed to adjust the final viscosities of the desired polyesters. However, such adjustment often leads to higher acid numbers (acid number ≥ 2.0 mg KOH/g) and decreased hydrolysis resistance of the obtained polyesters.

Therefore, the development of a bio-based succinic acid composition that has both a long shelf life and excellent aging stability, and meanwhile can decrease an acid number and a color value of a polyester to facilitate the preparation of a polyester with a high molecular weight, is an urgent problem to be solved in the art.

## SUMMARY

In view of the shortcomings of the prior art, the purpose of the present invention is to provide a bio-based succinic acid composition and a preparation method therefor. The bio-based succinic acid composition has a long shelf life, good aging stability, and a small change in b value after aging. Moreover, a polyester prepared from the bio-based succinic acid composition has a low acid number, a whiter color, and a higher molecular weight.

To achieve the purpose, the present invention adopts the following technical solutions.

In a first aspect, the present invention provides a bio-based succinic acid composition, where the bio-based succinic acid composition includes bio-based succinic acid and a readily oxidizable substance; and a content of the readily oxidizable substance in the bio-based succinic acid composition is ≤ 0.32 mL/g.

In the present invention, the presence of the readily oxidizable substance is conducive to improving the shelf life of the bio-based succinic acid. Meanwhile, by controlling the content of the readily oxidizable substance in the bio-based succinic acid composition and the b value of the bio-based succinic acid composition within specific ranges, the aging stability of the bio-based succinic acid composition can also be balanced while ensuring that the bio-based succinic acid composition has a long shelf life, so that the bio-based succinic acid composition has a small change in b value after aging. Moreover, using the bio-based succinic acid composition as a raw material to prepare the polyester is conducive to decreasing the acid number of the polyester and improving the intrinsic viscosity of the polyester, thus making the polyester less prone to yellowing, improving the production efficiency of the polyester, and avoiding the generation of a solid by-product.

In the present invention, the content of the readily oxidizable substance in the bio-based succinic acid composition is ≤ 0.32 mL/g, and for example, may be 0.001 mL/g, 0.005 mL/g, 0.01 mL/g, 0.02 mL/g, 0.04 mL/g, 0.06 mL/g, 0.08 mL/g, 0.1 mL/g, 0.12 mL/g, 0.14 mL/g, 0.16 mL/g, 0.18 mL/g, 0.2 mL/g, 0.22 mL/g, 0.24 mL/g, 0.26 mL/g, 0.28 mL/g, 0.30 mL/g, or a range between any of the above numerical ranges.

In the present invention, the content of the readily oxidizable substance is represented by a volume number of 0.1 mol/L potassium permanganate consumed by 1 g of the bio-based succinic acid composition, in the unit of mL/g.

Preferably, the content of the readily oxidizable substance in the bio-based succinic acid composition is 0.01-0.29 mL/g, more preferably 0.12-0.24 mL/g.

In the present invention, the content of the readily oxidizable substance is more preferably within the above defined range. When the content of the readily oxidizable substance is too low, the shelf life of the bio-based succinic acid is short; and when the content of the readily oxidizable substance is too high, the acid number of the obtained polyester is high, which is unfavorable for preparing the polyester with a high molecular weight. According to the provisions of GB/T 34686-2017, the content of the readily oxidizable substance in a qualified succinic acid product is ≤ 0.60 mL/g.

In the present invention, the readily oxidizable substance is a readily oxidizable substance generated during the preparation of succinic acid using a biomass resource as a raw material.

Preferably, the readily oxidizable substance includes cell debris, proteins, and amino acids directly derived from a fermentation solution, and also includes a nontoxic or low-toxic reducing agent, such as a sulfite or a thiosulfate, that may be selectively added to adjust the redox potential of a fermentation system as needed.

Preferably, according to standard GB/T 14190-2008 and using a CIE 1976 (L*a*b*) color space, a b value of the bio-based succinic acid composition is ≤ 4.2, and for example, may be 0.1, 0.2, 0.4, 0.6, 0.8, 1, 1.2, 1.4, 1.6, 1.8, 2, 2.2, 2.4, 2.6, 2.8, 3, 3.2, 3.4, 3.6, 3.8, 4, 4.2, or a range between any of the above numerical ranges; preferably, the b value is ≤ 2.5; and more preferably, the b value is 0.7-1.6.

Preferably, according to the standard GB/T 14190-2008 and using the CIE 1976 (L*a*b*) color space, a △b value of the bio-based succinic acid composition after aging at 150°C for 2 hours is ≤ 2.5; preferably, the △b value is ≤ 2.0; and more preferably, the △b value is ≤ 1.5.

In the present invention, the △b refers to a change in b value after aging, that is, a difference value between the b value of the bio-based succinic acid composition before aging and the b value of the bio-based succinic acid composition after aging.

In a second aspect, the present invention provides a method for preparing the bio-based succinic acid composition according to the first aspect, where the preparation method includes the following steps:

(1) using a biomass resource as a raw material to prepare and obtain a succinic acid stock solution; and
(2) purifying the succinic acid stock solution obtained in the step (1) to obtain the bio-based succinic acid composition, where

a method for the purifying includes treatment with an oxidizing agent solution, a mass of the oxidizing agent solution accounts for 18-50 wt% of a mass of a solution to be treated with the oxidizing agent solution, and a concentration of the oxidizing agent solution is 0.02-0.07 mol/L;
or, the preparation method includes the following steps: (S) subjecting bio-based succinic acid with a readily oxidizable substance content of ≥ 0.35 mL/g to recrystallization and filtration to obtain the bio-based succinic acid composition.

In the present invention, in the preparation method, by adding a specific content of the oxidizing agent solution for treatment or recrystallizing the bio-based succinic acid with a lower readily oxidizable substance content, the bio-based succinic acid with a specific content of the readily oxidizable substance can be obtained.

In the present invention, the solution to be treated with the oxidizing solution may be the succinic acid stock solution, and may also be a solution obtained after other purification treatments (such as neutralization with a neutralizing agent, adsorption by an ion exchange resin, and decolorization with activated carbon) of the succinic acid stock solution. The 18-50 wt% refers to a total mass of the oxidizing agent solution including a solvent.

In the present invention, the mass of the oxidizing agent solution accounts for 18-50 wt% of the mass of the solution to be treated with the oxidizing agent solution, and for example, may be 18 wt%, 19 wt%, 20 wt%, 21 wt%, 22 wt%, 23 wt%, 24 wt%, 25 wt%, 26 wt%, 27 wt%, 28 wt%, 29 wt%, 30 wt%, 31 wt%, 32 wt%, 33 wt%, 34 wt%, 35 wt%, 36 wt%, 37 wt%, 38 wt%, 39 wt%, 40 wt%, 42 wt%, 44 wt%, 45 wt%, 46 wt%, 48 wt%, 50 wt%, or a range between any of the above numerical ranges; and the concentration of the oxidizing agent solution is 0.02-0.07 mol/L, and for example, may be 0.02 mol/L, 0.03 mol/L, 0.04 mol/L, 0.05 mol/L, 0.06 mol/L, 0.07 mol/L, or a range between any of the above numerical ranges.

Preferably, the mass of the oxidizing agent solution accounts for 20-40 wt% of the mass of the solution to be treated with the oxidizing agent solution, more preferably 25-35 wt%.

In the present invention, the content of the oxidizing agent solution within the above range can not only ensure a lower content of the readily oxidizable substance in the obtained bio-based succinic acid composition, but also avoid an increase in the cost.

Preferably, the oxidizing agent solution includes at least one of an aqueous solution of potassium permanganate, an aqueous solution of hydrogen peroxide, and a chloric acid solution, more preferably the aqueous solution of potassium permanganate and/or the chloric acid solution.

Preferably, a temperature of the treatment with the oxidizing agent solution is 60-90°C, and for example, may be 60°C, 62°C, 64°C, 66°C, 68°C, 70°C, 72°C, 74°C, 76°C, 78°C, 80°C, 82°C, 84°C, 85°C, 90°C, or a range between any of the above numerical values; and a time is 35-165 min, and for example, may be 35 min, 40 min, 45 min, 50 min, 60 min, 70 min, 80 min, 90 min, 100 min, 110 min, 120 min, 130 min, 140 min, 150 min, 160 min, 165 min, or a range between any of the above numerical values.

Preferably, a mass concentration of succinic acid in the succinic acid stock solution in the step (1) is 70-95 g/L, and for example, may be 70 g/L, 72 g/L, 75 g/L, 78 g/L, 80 g/L, 82 g/L, 85 g/L, 88 g/L, 90 g/L, 92 g/L, 95 g/L, or a range between any of the above numerical values.

Preferably, the biomass resource includes a plant resource and/or an animal resource, preferably the plant resource. In the present invention, the plant resource refers to a biomass resource capable of converting light energy of the sun into the form of starch or cellulose and storing the same through photosynthesis; the animal resource refers to a biomass resource that grows and develops by preying on plant bodies; and the plant animal or the animal resource further includes a product obtained by processing the plant animal or the animal resource.

In the present invention, exemplarily, the plant resource includes, but is not limited to, wood, rice straw, rice husk, rice bran, aged rice, corn, sugarcane, cassava, corn straw, cassava starch residue, bagasse, plant oil residue, buckwheat, soybean, food waste, and the like.

In the present invention, the preparation method further includes converting the biomass resource into a carbon source, and then using the carbon source as a raw material to prepare and obtain the succinic acid stock solution. A method for converting the biomass resource into the carbon source includes, but is not limited to, chemical treatment, physical treatment, biological treatment, and the like. Exemplarily, the chemical treatment may adopt acid treatment (for example, treatment with strong acids, such as sulfuric acid, nitric acid, hydrochloric acid, and phosphoric acid), alkali treatment, ammonia freezing, distillation and explosion treatment, solvent extraction treatment, supercritical fluid treatment, oxidizing agent treatment, and the like. The physical treatment may be crushing treatment, distillation and explosion treatment, microwave treatment, electron ray irradiation treatment, and the like. The biological treatment may be microbial treatment, enzymatic treatment, and the like.

In the present invention, the carbon source exemplarily includes, but is not limited to, hexoses, such as glucose, mannose, galactose, fructose, sorbitol, and tagatose; pentoses, such as arabinose, xylose, ribose, xylulose, and ribulose; and disaccharides or polysaccharides, such as pentosan, sucrose, starch, and cellulose. The carbon source preferably includes the glucose, the fructose, and the xylose, particularly preferably the glucose.

Preferably, a method for the preparing in the step (1) includes a microbial fermentation method and/or a chemical conversion method, preferably the microbial fermentation method.

In the present invention, a microorganism used in the microbial fermentation method is capable of producing a dicarboxylic acid, and exemplarily includes: anaerobic bacteria, facultative anaerobic bacteria, aerobic bacteria, and the like; the anaerobic bacteria may be, for example, of the genus *Anaerobiospirillum* (US5143833A); the facultative anaerobic bacteria may be, for example, of the genus *Actinobacillus* (US5504004A), the genus *Escherichia* (US5770435A), and the like; and the aerobic bacteria may be, for example, of the genus *Corynebacterium* (JPH0 11-113588 communique or CN103183813A), where these documents are incorporated herein by reference.

In the present invention, the succinic acid stock solution prepared and obtained using the biomass resource as a raw material in the step (1) may be prepared by a conventional method in the art; and exemplarily, in the preparation method, the succinic acid stock solution prepared and obtained using the biomass resource as a raw material in the step (1) is prepared by a conventional method in the art, and for example, may refer to a method in CN118459331A.

Preferably, the method for the purifying in the step (2) further includes any one or a combination of at least two of filtration, treatment with a neutralizing agent, treatment with activated carbon, and treatment with an ion exchange resin.

In the present invention, the neutralizing agent includes ammonia, ammonium carbonate, urea, alkali metal hydroxides, alkaline earth metal hydroxides, alkali metal carbonates, alkaline earth metal carbonates, and the like. At least one of the ammonia, the ammonium carbonate, or the urea is preferred. The alkali metal hydroxides or alkaline earth metal hydroxides include, but are not limited to, one or a mixture of more of NaOH, KOH, $Ca(OH)_2$, and $Mg(OH)_2$; and the alkali metal carbonates or alkaline earth metal carbonates include, but are not limited to, one or a mixture of more of $Na_2CO_3$, $K_2CO_3$, $CaCO_3$, and $MgCO_3$.

Preferably, a time of the treatment with the neutralizing agent is 60-120 min; and a mass concentration of the neutralizing agent is 18-42 wt%, and for example, may be 20 wt%, 21 wt%, 22 wt%, 23 wt%, 24 wt%, 25 wt%, 26 wt%, 27 wt%, 28 wt%, 29 wt%, 30 wt%, 31 wt%, 32 wt%, 33 wt%, 34 wt%, 35 wt%, 36 wt%, 37 wt%, 38 wt%, 39 wt%, 40 wt%, or a range between any of the above numerical values.

Preferably, the preparation method further includes steps of filtration and washing after the treatment with the neutralizing agent.

Preferably, based on a volume of 1 L of a solution to be treated with the activated carbon, a mass of the activated carbon is 5-8 g, and for example, may be 5 g, 5.2 g, 5.5 g, 5.8 g, 6 g, 6.2 g, 6.5 g, 6.8 g, 7 g, 7.2 g, 7.5 g, 7.8 g, 8 g, or a range between any of the above numerical values.

In the present invention, the solution to be treated with the activated carbon may be the succinic acid stock solution, and may also be a solution obtained after other purification treatments, such as filtration, neutralization with a neutralizing agent, and adsorption by an ion exchange resin.

Preferably, the ion exchange resin includes a cation exchange resin and/or an anion exchange resin.

In the present invention, treatment with the cation exchange resin includes: allowing a solution to be treated with the ion exchange resin to pass through a column filled with the cation exchange resin, performing elution with water, and collecting an effluent, where a mass of the water is 2-5 times that of the solution to be treated with the ion exchange resin, and for example, may be 2 times, 2.5 times, 3 times, 3.5 times, 4 times, 4.5 times, 5 times, or a range between any of the above numerical values.

In the present invention, treatment with the anion exchange resin includes: allowing a solution to be treated with the ion exchange resin to pass through a column filled with the anion exchange resin, performing elution with water, and collecting an effluent, where a mass of the water is 2-5 times that of the solution to be treated with the ion exchange resin, and for example, may be 2 times, 2.5 times, 3 times, 3.5 times, 4 times, 4.5 times, 5 times, or a range between any of the above numerical values.

Preferably, after the purifying in the step (2), the preparation method further includes a step of reduced-pressure distillation and/or cooling crystallization.

In the present invention, a temperature of the reduced-pressure distillation is 65-75°C, a pressure is -0.07 - -0.1 MPa, and the distillation is stopped when the content of succinic acid is greater than 15 wt%; and the concentrated succinic acid solution is subjected to the cooling crystallization at a low temperature ($\leq 8$°C) to obtain the bio-based succinic acid composition.

In the present invention, the bio-based succinic acid with the readily oxidizable substance content of $\geq 0.35$ mL/g in the step (S) may be commercially available or prepared by a conventional method.

Preferably, a method for the recrystallization in the step (S) includes: mixing the bio-based succinic acid with a solvent at 60-100°C (for example, may be 60°C, 65°C, 70°C, 75°C, 80°C, 85°C, 90°C, 95°C, 100°C, or a range between any of the above numerical values), and then cooling to 15-25°C (for example, may be 15°C, 16°C, 17°C, 18°C, 19°C, 20°C, 22°C, 24°C, 25°C, or a range between any of the above numerical values) at a rate of 5-15°C/h (for example, may be 5°C/h, 6°C/h, 8°C/h, 10°C/h, 12°C/h, 14°C/h, 15°C/h, or a range between any of the above numerical values) for recrystallization.

Preferably, the solvent in the recrystallization includes water.

Preferably, a number of times of the recrystallization in the step (S) is $\geq 1$ time, and for example, may be 1 time, 2 times, 3 times, 4 times, 5 times, 6 times, 7 times, 8 times, 9 times, 10 times, or a range between any of the above numerical values.

In the present invention, when the bio-based succinic acid composition is prepared by a recrystallization method, the content of the readily oxidizable substance in the bio-based succinic acid is $\leq 0.6$ mL/g.

As a preferred technical solution of the present invention, the preparation method includes the following steps:

(1) using the biomass resource as the raw material to prepare and obtain the succinic acid stock solution with a succinic acid content of 70-95 g/L;

(2) subjecting the succinic acid stock solution obtained in the step (1) to first filtration, then adding a neutralizing agent thereinto for mixing for 60-120 min, and performing second filtration to obtain a filtrate A with a succinic acid content of 65-88 g/L;

(3) mixing the filtrate A obtained in the step (2) with an oxidizing agent solution at 60-90°C for 35-165 min, and then adding activated carbon thereinto for mixing for 30-80 min to obtain a filtrate B with a succinic acid content of 50-65 g/L;

(4) subjecting the filtrate B obtained in the step (3) to treatment with a cation exchange resin and an anion exchange resin to obtain a filtrate C, where there is no requirement for the order of the treatment with the cation exchange resin and the treatment with the anion exchange resin, the treatment with the cation exchange resin and the treatment with the anion exchange resin may be sequentially adopted, or the treatment with the anion exchange resin and the treatment with the cation exchange resin are sequentially adopted; where the filtrate B is allowed to pass once or more times through a column filled with one or more cation exchange resins/anion exchange resins, water (with a mass 2-5 times that of the filtrate B) is added for elution, and an effluent is collected to obtain the filtrate C with a succinic acid content of 10-30 g/L; where

a mass of the oxidizing agent solution accounts for 18-50 wt% of a mass of the filtrate A, a concentration of the oxidizing agent solution is 0.02-0.07 mol/L, and based on a volume of 1 L of the filtrate A, a mass of the activated carbon is 5-8 g; and

(5) subjecting the filtrate C obtained in the step (4) to reduced-pressure distillation and cooling crystallization to obtain the bio-based succinic acid composition;

or, the preparation method includes the following steps:

(S) mixing and dissolving the bio-based succinic acid with the readily oxidizable substance content of ≥ 0.35 mL/g and a solvent at 60-100°C, then cooling to 15-25°C at a rate of 5-15°C/h for recrystallization, performing filtration, and repeating the aforementioned steps at least once to obtain the bio-based succinic acid composition.

In a third aspect, the present invention provides a polyester, where raw materials for preparing the polyester include the bio-based succinic acid composition described in the first aspect.

The polyester produced using the specific bio-based succinic acid composition of the present invention as a raw material has a lower acid number and a higher molecular weight.

Preferably, the polyester includes the following components:

component A:

based on a total molar amount of 100 mol% of the component A, being a dicarboxylic acid compound including the following components:
a1, being 65-100 mol% of the bio-based succinic acid composition described in the first aspect and/or a derivative of an ester thereof, and
a2, being 0-35 mol% of adipic acid and/or a derivative of an ester thereof;
and component B: being 1,4-butanediol with a molar amount at least equal to that of the component A.

In the present invention, a molar ratio of the component B to the component A is (1-3):1, and for example, may be 1:1, 1.2:1, 1.5:1, 1.8:1, 2:1, 2.2:1, 2.5:1, 2.8:1, 3:1, or a range between any of the above numerical values, more preferably (1-1.6):1.

In the present invention, the phrase "the polyester includes the following components" means that a molecular structure of the polyester includes a structural unit derived from the component A and a structural unit derived from the component B; and the phrase "a dicarboxylic acid compound including the following components" means that in the molecular structure of the polyester, a dicarboxylic acid structural unit part includes a structural unit derived from the component a1 and a structural unit derived from the component a2.

In the present invention, the 65-100 mol%, for example, may be 65 mol%, 66 mol%, 68 mol%, 70 mol%, 72 mol%, 74 mol%, 76 mol%, 78 mol%, 80 mol%, 82 mol%, 84 mol%, 86 mol%, 88 mol%, 90 mol%, 92 mol%, 94 mol%, 96 mol%, 98 mol%, 100 mol%, or a range between any of the above numerical values.

In the present invention, the 0-35 mol%, for example, may be 0 mol%, 2 mol%, 4 mol%, 6 mol%, 8 mol%, 10 mol%, 12 mol%, 14 mol%, 16 mol%, 18 mol%, 20 mol%, 22 mol%, 24 mol%, 26 mol%, 28 mol%, 30 mol%, 32 mol%, 34 mol%, 35 mol%, or a range between any of the above numerical values.

Preferably, based on the total molar amount of 100 mol% of the component A, the dicarboxylic acid compound includes the following components:

a1, being 72-82 mol% of the bio-based succinic acid composition described in the first aspect and/or a derivative of an ester thereof, and
a2, being 18-28 mol% of the adipic acid and/or a derivative of an ester thereof.

In the present invention, a derivative of a succinic acid ester includes an alkyl succinate; exemplarily, the alkyl succinate may be at least one of dimethyl succinate, diethyl succinate, di-n-propyl succinate, diisopropyl succinate, di-n-butyl succinate, diisobutyl succinate, di-tert-butyl succinate, di-n-pentyl succinate, diisopentyl succinate, and di-n-hexyl succinate; and the alkyl succinate may be an alkyl ester formed from succinic acid or an alkyl ester formed from succinic anhydride, and dimethyl succinate formed from succinic anhydride is preferably used.

In the present invention, a derivative of an adipic acid ester includes an alkyl adipate; exemplarily, the alkyl adipate may be at least one of dimethyl adipate, diethyl adipate, di-n-propyl adipate, diisopropyl adipate, di-n-butyl adipate, diisobutyl adipate, di-tert-butyl adipate, di-n-pentyl adipate, diisopentyl adipate, and di-n-hexyl adipate; and the alkyl adipate may be an alkyl ester formed from adipic acid or an alkyl ester formed from adipic anhydride.

In the present invention, a dicarboxylic acid or a derivative of an ester thereof may be used separately or used in the form of a mixture of two or more.

In the present invention, the polyester may be prepared by a conventional technical method in the art. Exemplarily, the method includes the following steps:
allowing the component A and the component B to undergo an esterification reaction at a temperature of 140-220°C and a pressure of 0.5-2 bar for 1-6 h to obtain an esterification product; allowing the esterification product to undergo a pre-polycondensation reaction at a temperature of 220-270°C and a pressure of 0.1-1 bar for 60-140 min to obtain a pre-polycondensation product; and then allowing the pre-polycondensation product to undergo a polycondensation reaction at a temperature of 230-280°C and a pressure of 1-5 mbar for 60-100 min, and performing pelletizing and drying to obtain the polyester.

In the present invention, raw materials for the esterification reaction further include a cross-linking agent, where the cross-linking agent includes at least one of tartaric acid, citric acid, malic acid, trimethylolpropane, trimethylolethane, pentaerythritol, polyether triol, glycerol, 1,3,5-benzenetricarboxylic acid, 1,2,4-benzenetricarboxylic acid, 1,2,4-benzenetricarboxylic anhydride, 1,2,4,5-benzenetetracarboxylic acid, or pyromellitic dianhydride; and based on a mass of 100 wt% of a finished product of the polyester, a mass percentage content of the cross-linking agent is 0.05-1 wt%.

In the present invention, the pre-polycondensation reaction further includes a catalyst; the catalyst may be a tin compound, an antimony compound, a cobalt compound, a lead compound, a zinc compound, an aluminum compound, or a titanium compound, more preferably the zinc compound, the aluminum compound, or the titanium compound, and most preferably the titanium compound; the titanium compound may be tetrabutyl titanate or tetraisopropyl titanate; and a total mass of the catalyst is 0.001-1 wt% of a mass of a polycondensation product.

Preferably, according to standard DIN EN 12634-1998, an acid number of the polyester is $\leq$ 1.20 mg KOH/g.

Preferably, according to standard GB/T 14190-2008 and using a CIE 1976 (L*a*b*) color system, an a value of the polyester is $\leq$ 1.5, further preferably $\leq$ 1.0, and more preferably $\leq$ 0.8.

Preferably, an intrinsic viscosity of the polyester determined according to GB/T 17931-1999 is $\geq$ 1.40 dL/g, further preferably $\geq$ 1.45 dL/g, and more preferably $\geq$ 1.60 dL/g.

The polyester of the present invention also has biodegradability.

For the present invention, if a substance or a substance mixture shows, as defined in DIN EN 13432, a biodegradation percentage degree of at least 90%, the substance or the substance mixture has the characteristic of "biodegradability".

Biodegradation usually leads to decomposition of a polyester or a polyester mixture within a reasonable inspection period. Degradation may occur through an enzymatic, hydrolytic, or oxidative pathway, and/or through exposure to electromagnetic radiation such as ultraviolet radiation, and is most commonly caused by exposure to microorganisms such as bacteria, yeast, fungi, or algae. By mixing the polyester with compost and storing the same for a specific period of time, the biodegradability may be quantified. For example, according to DIN EN 13432, during composting, air free of $CO_2$ is introduced into mature compost, and the compost is allowed to undergo a specific temperature process. Herein, the biodegradability is defined as a biodegradation percentage degree represented by a ratio of a net amount of $CO_2$ released by a sample (after subtracting an amount of $CO_2$ released by compost without the sample) to a maximum amount of $CO_2$ that can be released by the sample (calculated based on a carbon content in the sample).

Additionally, other methods for determining the biodegradability are described in ASTM D5338 and ASTM D6400.

The numerical ranges in the present invention include not only the above-mentioned enumerated point values but also any point values within the above-mentioned numerical ranges that are not enumerated, and due to space limitations and for the sake of simplicity, all specific point values included within the ranges are not exhaustively enumerated in the present invention.

Compared with the prior art, the present invention has the following beneficial effects.

The bio-based succinic acid composition provided by the present invention includes a specific content of the readily oxidizable substance, which can ensure that the bio-based succinic acid composition has a small change in b value before and after aging without affecting the production efficiency and process of the polyester, and has a long shelf life. The polyester obtained using the bio-based succinic acid composition as a raw material has a lower acid number, a whiter color, and a higher molecular weight.

## DETAILED DESCRIPTION OF EMBODIMENTS

The technical solutions of the present invention are further described below through specific embodiments. Those skilled in the art should understand that examples described are merely to facilitate an understanding of the present invention and should not be regarded as specific limitations to the present invention.

Unless otherwise specified, raw materials used in the present invention are as follows:

1,4-butanediol: purchased from Xinjiang Markorchem Co., Ltd.;
glycerol: purchased from Aladdin;
commercially available bio-based succinic acid with a first-grade specification: purchased from Shandong Landian Biotechnology Co., Ltd., with a readily oxidizable substance content of 0.47 mL/g; and

tetrabutyl titanate: purchased from Jianyi Chemical Import and Export Co., Ltd.

In the present invention, the content of the readily oxidizable substance is tested by referring to GB/T 34686-2017. A specific test method is as follows:

1) sample determination

weighing 1.0 g of a sample accurately to 0.0001 g, separately adding 30 mL of water free of carbon dioxide and 30 mL of a sulfuric acid solution (with a mass fraction of 20%), and titrating the solution with a 0.1 mol/L standard titration solution of potassium permanganate until the solution turns pink and remains for 3 min without complete fading to reach an endpoint, where throughout the titration, a temperature of the sample solution is not lower than 55°C; and

2) result calculation

expressing the readily oxidizable substance X as a volume of the 0.1 mol/L standard titration solution of potassium permanganate consumed by per gram of the sample, in the unit of milliliter per gram (mL/g). A calculation formula is as follows:

$$X = \frac{c \times V}{m \times c_0} \; ;$$

where,

V refers to a volume of the 0.1 mol/L standard titration solution of potassium permanganate consumed, in the unit of milliliter (mL);
c refers to an actual concentration of the standard titration solution of potassium permanganate, in the unit of mole per liter (mol/L);
m refers to a mass of the sample, in the unit of gram (g); and
$c_0$ refers to a theoretical concentration of the 0.1 potassium permanganate, in the unit of mole per liter (mol/L).

An arithmetic mean value of two parallel measurement results is taken as a result, and a difference between the two parallel measurement results does not exceed 5% of the mean value.

In the present invention, a b value of a bio-based succinic acid composition before and after aging is tested according to standard GB/T 14190-2008 and using a CIE 1976 (L*a*b*) color space, where an aging method specifically includes: weighing 20±0.5 g of a sample into a weighing flask, placing the weighing flask in a blast drying oven at a constant temperature of 150°C for baking for 2 h, placing the weighing flask in a dryer for cooling for 20±2 min after aging, testing the b value of the aged bio-based succinic acid composition according to the standard GB/T 14190-2008 and using the CIE 1976 (L*a*b*) color space, and calculating a change amount of the b value (△b value).

In the present invention, the purity of a bio-based succinic acid composition (i.e., the mass percentage content of bio-based succinic acid in the bio-based succinic acid composition) is determined according to a detailed method specified in 4.3 of standard GB/T 34686-2017.

In the present invention, in a preparation method, the concentration of succinic acid in an obtained succinic acid fermentation solution and a filtrate is tested by liquid chromatography, and a specific test method refers to a method disclosed in CN118459331A.

Example 1

This example provides a bio-based succinic acid composition, which includes bio-based succinic acid and a readily oxidizable substance, where a content of the readily oxidizable substance in the bio-based succinic acid composition is 0.18 mL/g.

This example provides a method for preparing the bio-based succinic acid composition, which specifically includes the following steps:

(1) using a biomass resource as a raw material to prepare and obtain a succinic acid fermentation solution with a succinic acid content of 81 g/L by a microbial fermentation method, where the fermentation solution is prepared by a conventional method in the art, and for example, may be prepared according to a method in CN118459331A;
(2) filtering the succinic acid fermentation solution obtained in the step (1), adding 3 g of a $Na_2CO_3$ solution with a mass concentration of 30% into a resulting filtrate based on a volume of 1 L of the filtrate (with a succinic acid content of 85.1 g/L), carrying out a reaction in a stirring state for 80 min, and then performing filtration to obtain a filtrate A;

(3) adding a 0.05 mol/L aqueous solution of $KMnO_4$ with a mass of 40 wt% into the filtrate A obtained in the step (2), heating up to 85°C, stirring the solution to carry out a reaction for 160 min, then adding activated carbon for decolorization for 60 min in a use amount of 7 g based on a volume of 1 L of the filtrate A, and performing filtration to obtain a filtrate B;

(4) allowing the filtrate B obtained in the step (3) to sequentially pass through a cation exchange resin column and an anion exchange resin column, performing elution with water with a weight 5 times that of the filtrate B, repeating washing 4 times, and collecting an effluent to obtain a filtrate C with a succinic acid content of 16.2 g/L; and

(5) subjecting the filtrate C obtained in the step (4) to reduced-pressure distillation treatment at a distillation temperature of 70°C and a pressure of -0.07 - - 0.1 MPa, sampling for liquid chromatography testing and moisture testing until the succinic acid content is greater than 15 wt%, terminating the distillation, and subjecting the concentrated succinic acid solution to cooling crystallization at a low temperature (4°C) to obtain a white succinic acid crystal, thereby obtaining the bio-based succinic acid composition.

Example 2

This example provides a bio-based succinic acid composition, which includes bio-based succinic acid and a readily oxidizable substance, where a content of the readily oxidizable substance in the bio-based succinic acid composition is 0.23 mL/g.

This example provides a method for preparing the bio-based succinic acid composition, which specifically includes the following steps:

(1) using a biomass resource as a raw material to prepare and obtain a succinic acid stock solution by a microbial fermentation method, where the preparation method is the same as that in Example 1;

(2) filtering the succinic acid fermentation solution obtained in the step (1), adding 2 g of a $Na_2CO_3$ solution with a mass concentration of 25% into a resulting filtrate based on a volume of 1 L of the filtrate (with a succinic acid content of 83.5 g/L), carrying out a reaction in a stirring state for 70 min, and then performing filtration to obtain a filtrate A;

(3) adding a 0.05 mol/L $KMnO_4$ solution with a mass of 30% into the filtrate A obtained in the step (2), heating up to 85°C, stirring the solution to carry out a reaction for 160 min, then adding activated carbon for decolorization for 60 min in a use amount of 7 g based on a volume of 1 L of the filtrate A, and performing filtration to obtain a filtrate B;

(4) allowing the filtrate B obtained in the step (2) to sequentially pass through a cation exchange resin column and an anion exchange resin column, performing elution with water with a weight 4 times that of the filtrate B, repeating washing 3 times, and collecting an effluent to obtain a filtrate C with a succinic acid content of 19.8 g/L; and

(5) subjecting the filtrate C obtained in the step (4) to reduced-pressure distillation treatment at a distillation temperature of 70°C and a pressure of -0.07 - - 0.1 MPa, terminating the distillation when the succinic acid content is greater than 15 wt%, and subjecting the concentrated succinic acid solution to cooling crystallization at a low temperature (4°C) to obtain a white succinic acid crystal, thereby obtaining the bio-based succinic acid composition.

Example 3

This example provides a bio-based succinic acid composition, which includes bio-based succinic acid and a readily oxidizable substance, where a mass of the readily oxidizable substance in the bio-based succinic acid composition is 0.28 mL/g.

This example provides a method for preparing the bio-based succinic acid composition, which specifically includes the following steps:

(1) using a biomass resource as a raw material to prepare and obtain a succinic acid stock solution by a microbial fermentation method, where the preparation method is the same as that in Example 1;

(2) filtering the succinic acid fermentation solution obtained in the step (1), adding 3 g of a $Na_2CO_3$ solution with a mass concentration of 30% into a resulting filtrate based on a volume of 1 L of the filtrate (with a succinic acid content of 84.2 g/L), carrying out a reaction in a stirring state for 90 min, and then performing filtration to obtain a filtrate A;

(3) adding a 0.05 mol/L $KMnO_4$ solution with a mass of 20% into the filtrate A obtained in the step (2), heating up to 80°C, stirring the solution to carry out a reaction for 40 min, then adding activated carbon for decolorization for 60 min in a use amount of 6 g based on a volume of 1 L of the filtrate A, and performing filtration to obtain a filtrate B;

(4) allowing the filtrate B obtained in the step (3) to sequentially pass through a cation exchange resin column and an anion exchange resin column, performing elution with water with a weight 4 times that of the filtrate B, repeating washing 4 times, and collecting an effluent to obtain a filtrate C with a succinic acid content of 20.3 g/L; and

(5) subjecting the filtrate C obtained in the step (4) to reduced-pressure distillation treatment at a distillation temperature of 70°C and a pressure of -0.07 - - 0.1 MPa, terminating the distillation when the succinic acid content is greater than 15 wt%, and subjecting the concentrated succinic acid solution to cooling crystallization at a low temperature (4°C) to obtain a white succinic acid crystal, thereby obtaining the bio-based succinic acid composition.

Example 4

This example provides a bio-based succinic acid composition, where the only difference from Example 1 is that in the preparation method, the "0.05 mol/L $KMnO_4$ solution with a mass of 40%" in the step (3) is adjusted to "a 0.05 mol/L $KMnO_4$ solution with a mass of 45%". Others in the preparation method are all the same as those in Example 1.

Example 5

This example provides a bio-based succinic acid composition and a preparation method therefor. The preparation method specifically includes the following steps:

(1) adding commercially available bio-based succinic acid at a concentration of 40 g/100 mL into deionized water under room temperature conditions (23±2°C), and then heating the deionized water solution containing the bio-based succinic acid to 85°C to completely dissolve the bio-based succinic acid to form an aqueous solution of bio-based succinic acid;
(2) cooling the aqueous solution of bio-based succinic acid obtained in the step (1) to 20±1°C at a cooling rate of 10°C/h to precipitate a bio-based succinic acid crystal;
(3) filtering the solution obtained in the step (2) to obtain a bio-based succinic acid product A; and
(4) allowing the bio-based succinic acid product A obtained in the step (3) to undergo the above steps (1)-(3) repeatedly to obtain a target bio-based succinic acid product, that is, the bio-based succinic acid composition with a low readily oxidizable substance content.

Example 6

This example provides a bio-based succinic acid composition, where the only difference from Example 1 is that in the preparation method, the $KMnO_4$ in the step (3) is replaced with a 10% aqueous solution of $H_2O_2$ with a mass of 40% that of the filtrate B. Others in the preparation method are all the same as those in Example 1.

Comparative Example 1

This comparative example provides a bio-based succinic acid composition, where the only difference from Example 1 is that in the preparation method, the step (3) is omitted. Others in the preparation method are all the same as those in Example 1.

Comparative Example 2

This comparative example provides a bio-based succinic acid composition, where the only difference from Example 1 is that in the preparation method, the "0.05 mol/L $KMnO_4$ solution with a mass of 40%" in the step (3) is adjusted to "a 0.05 mol/L $KMnO_4$ solution with a mass of 15%". Others in the preparation method are all the same as those in Example 1.

Test results of the mass percentage content of succinic acid, the content of the readily oxidizable substance, and b values before and after aging of the bio-based succinic acid compositions provided in Examples 1-6 and Comparative Examples 1 and 2 are shown in Table 1. Remainders in the bio-based succinic acid compositions include water and other impurity acids remaining in the process of preparing the succinic acid.

A method for testing the shelf life of a bio-based succinic acid composition includes: placing the bio-based succinic acid composition in a blast drying oven at 80°C to carry out a thermal oxidation baking experiment, testing the purity of the bio-based succinic acid composition every 4 hours, and when the purity of the bio-based succinic acid composition is lower than 99.0%, subtracting 4 hours from the corresponding time to obtain the shelf life of succinic acid. For example, when the purity of the succinic acid is lower than 99.0% and the time is 80 hours, the shelf life is 76 hours.

Table 1

| | Mass percentage content of bio-based succinic acid (wt%) | Content of a readily oxidizable substance (mL/g) | b value (before aging) | b value (after aging) | △b value | Product shelf life (h) |
|---|---|---|---|---|---|---|
| Example 1 | 99.82 | 0.18 | 0.8 | 1.9 | 1.1 | 68 |
| Example 2 | 99.79 | 0.23 | 1.9 | 3.6 | 1.7 | 76 |
| Example 3 | 99.71 | 0.28 | 2.7 | 4.8 | 2.1 | 84 |
| Example 4 | 99.67 | 0.11 | 1.0 | 2.6 | 1.6 | 44 |
| Example 5 | 99.84 | 0.18 | 1.5 | 2.9 | 1.4 | 68 |
| Example 6 | 99.64 | 0.30 | 3.4 | 5.8 | 2.4 | 80 |
| Comparative Example 1 | 99.50 | 0.50 | 4.8 | 7.9 | 3.1 | 84 |
| Comparative Example 2 | 99.53 | 0.42 | 4.4 | 7.2 | 2.8 | 84 |

It can be seen from Table 1 that by controlling the content of the readily oxidizable substance in the bio-based succinic acid composition provided by the present invention within a specific range, the bio-based succinic acid can have both aging stability (low △b value) and a long product shelf life.

Application Example 1

This application example provides a polyester. A method for preparing the polyester includes:

S1: physically mixing 187.9 kg of the bio-based succinic acid composition provided in Example 1, 205 kg of 1,4-butanediol, and 0.85 kg of glycerol, and after the mixing is completed, transferring a resulting mixture into a multi-stage stirring tank cascade reactor to allow the reaction mixture to carry out an esterification reaction at a temperature of 180°C and a pressure of 1.0 bar for 3 h to obtain an esterification product with an intrinsic viscosity of 0.08 dL/g;
S2: transferring the esterification product obtained in the step S1 into a vertical reactor with a stirrer, adding 0.10 kg of tetrabutyl titanate, and allowing the reaction mixture to carry out a pre-polycondensation reaction at 240°C and at an internal reactor pressure of 0.35 bar for 120 min to obtain a pre-polycondensation product with an intrinsic viscosity of 0.42 dL/g; and
S3: transferring the pre-polycondensation product obtained in the step S2 into a horizontal reactor with a stirrer to carry out a polycondensation reaction at 243°C and at a pressure of 2.0 mbar for 85 min, and performing pelletizing and drying to obtain the polyester.

Application Examples 2-6 and Comparative Application Examples 1 and 2

Application Examples 2-6 and Comparative Application Examples 1 and 2 respectively provide a polyester, where the only difference from Application Example 1 is that the bio-based succinic acid composition is provided by Examples 2-6 and Comparative Examples 1 and 2, respectively. Other raw materials, use amounts, and preparation methods are all the same as those in Application Example 1.

Performance tests

(1) Intrinsic viscosity: According to a method specified in GB/T 17931-1999, the intrinsic viscosity was determined in a phenol/o-dichlorobenzene solution with a weight ratio of 1:1 in a thermostatic water bath at 25±0.05°C.
(2) Acid number: The acid number AN (mg KOH/g) of a sample was determined according to the standard DIN EN 12634-1998.

A solvent mixture used includes: 1 part by volume of dimethyl sulfoxide, 8 parts by volume of isopropanol, and 7 parts by volume of toluene, where a total volume of the solvent mixture was 150 mL.
A specific method includes that according to the provisions of the standard DIN EN 12634-1998, samples were pre-titrated to determine appropriate sample masses and ensure that a volume of a titration solution consumed was 2-3 mL. The samples were added into the solvent mixture and heated to 70-85°C to ensure that all the samples were completely dissolved into clear solutions, and during the titration, the temperatures of the solutions were maintained at 65-75°C to avoid precipitation of the samples. If appropriate, tetrabutylammonium hydroxide was selected as the titration solution while avoiding the use of highly toxic tetramethylammonium hydroxide. Meanwhile, to prevent the solvent mixture from absorbing $CO_2$ in the air and affecting the volume of the titration solution consumed by a blank solvent, when the volume of the titration solution consumed by the blank solvent was tested, the blank solvent was pre-treated in the same test operation process as the samples. For example, the blank solvent was subjected to heating treatment at the same time and temperature, and then the blank solvent was titrated.

(3) Color value: According to the standard GB/T 14190-2008, an a value of a polyester was tested using the CIE 1976 (L*a*b*) color space.

Specific test results are shown in Table 2.

Table 2

|  | Acid number (mg KOH/g) | a value | Intrinsic viscosity (dL/g) |
|---|---|---|---|
| Application Example 1 | 0.85 | -0.4 | 1.67 |
| Application Example 2 | 1.08 | 0.6 | 1.61 |
| Application Example 3 | 1.13 | 0.9 | 1.57 |
| Application Example 4 | 1.04 | 0.1 | 1.63 |
| Application Example 5 | 0.94 | -0.1 | 1.65 |
| Application Example 6 | 1.18 | 1.2 | 1.46 |
| Comparative Application Example 1 | 2.11 | 2.1 | 1.32 |
| Comparative Application Example 2 | 1.73 | 1.8 | 1.37 |

It can be seen from Table 2 that the polyester prepared and obtained using the specific bio-based succinic acid composition provided by the present invention as a raw material has a low acid number of $\leq 1.18$ mg KOH/g, an intrinsic viscosity of $\geq 1.46$ dL/g, a higher molecular weight, a color value (a) of $\leq 1.5$, and a whiter color.

From the comparison between application examples and comparative application examples, it can be seen that when the content of the readily oxidizable substance in the bio-based succinic acid composition is not within the specified range, a change in the b value before and after aging is too large, and the aging stability is poor; and the obtained polyester has a high acid number, a high color value, a low intrinsic viscosity, and a lower molecular weight.

The specific examples described above are to further describe the purposes, technical solutions, and beneficial effects of the present invention in detail. It should be understood that the above descriptions are only specific examples of the present invention and are not intended to limit the present invention, and any modifications, equivalent substitutions, improvements, and the like made within the spirit and principles of the present invention should all be included within the scope of protection of the present invention.

**Claims**

1. A bio-based succinic acid composition, comprising bio-based succinic acid and a readily oxidizable substance, wherein
   a content of the readily oxidizable substance in the bio-based succinic acid composition is $\leq 0.32$ mL/g.

2. The bio-based succinic acid composition according to claim 1, wherein the content of the readily oxidizable substance in the bio-based succinic acid composition is 0.01-0.29 mL/g.

3. The bio-based succinic acid composition according to claim 1, wherein according to standard GB/T 14190-2008 and using a CIE 1976 (L*a*b*) color space, a b value of the bio-based succinic acid composition is $\leq 4.2$; and
   according to the standard GB/T 14190-2008 and using the CIE 1976 (L*a*b*) color space, a $\triangle$b value of the bio-based succinic acid composition after aging at 150°C for 2 hours is $\leq 2.5$.

4. A method for preparing the bio-based succinic acid composition according to any one of claims 1-3, comprising the following steps:

(1) using a biomass resource as a raw material to prepare and obtain a succinic acid stock solution; and

(2) purifying the succinic acid stock solution obtained in the step (1) to obtain the bio-based succinic acid composition, wherein

a method for the purifying comprises treatment with an oxidizing agent solution, a mass of the oxidizing agent solution accounts for 18-50 wt% of a mass of a solution to be treated with the oxidizing agent solution, and a concentration of the oxidizing agent solution is 0.02-0.07 mol/L;

or,

the preparation method comprises the following steps:

(S) subjecting bio-based succinic acid with a readily oxidizable substance content of $\geq$ 0.35 mL/g to recrystallization and filtration to obtain the bio-based succinic acid composition.

5. The preparation method according to claim 4, wherein the mass of the oxidizing agent solution accounts for 20-40 wt% of the mass of the solution to be treated with the oxidizing agent solution;

the oxidizing agent solution comprises at least one of an aqueous solution of potassium permanganate, an aqueous solution of hydrogen peroxide, or a chloric acid solution;

a temperature of the treatment with the oxidizing agent solution is 60-90°C, and a time is 35-165 min; and

a mass concentration of succinic acid in the succinic acid stock solution in the step (1) is 70-95 g/L.

6. The preparation method according to claim 4, wherein the biomass resource in the step (1) comprises a plant resource and/or an animal resource;

a method for the preparing in the step (1) comprises a microbial fermentation method and/or a chemical conversion method;

the method for the purifying in the step (2) further comprises any one or a combination of at least two of filtration, treatment with a neutralizing agent, treatment with activated carbon, and treatment with an ion exchange resin;

a time of the treatment with the neutralizing agent is 60-120 min;

based on a volume of 1 L of a solution to be treated with the activated carbon, a mass of the activated carbon is 5-8 g;

the ion exchange resin comprises a cation exchange resin and/or an anion exchange resin;

after the purifying in the step (2), the preparation method further comprises a step of reduced-pressure distillation and/or cooling crystallization;

a method for the recrystallization in the step (S) comprises: mixing the bio-based succinic acid with a solvent at 60-100°C, and then cooling to 15-25°C at a rate of 5-15°C/h for recrystallization;

the solvent in the recrystallization comprises water; and

a number of times of the recrystallization in the step (S) is $\geq$ 1 time.

7. The preparation method according to claim 4, comprising the following steps:

(1) using the biomass resource as the raw material to prepare and obtain the succinic acid stock solution with a succinic acid content of 70-95 g/L;

(2) subjecting the succinic acid stock solution obtained in the step (1) to first filtration, then adding a neutralizing agent thereinto for mixing for 60-120 min, and performing second filtration to obtain a filtrate A with a succinic acid content of 65-88 g/L;

(3) mixing the filtrate A obtained in the step (2) with an oxidizing agent solution at 60-90°C for 35-165 min, and then adding activated carbon thereinto for mixing for 30-80 min to obtain a filtrate B with a succinic acid content of 50-65 g/L;

(4) treating the filtrate B obtained in the step (3) with a cation exchange resin and an anion exchange resin to obtain a filtrate C with a succinic acid content of 10-30 g/L, wherein

a mass of the oxidizing agent solution accounts for 18-50 wt% of a mass of the filtrate A, a concentration of the oxidizing agent solution is 0.02-0.07 mol/L, and based on a volume of 1 L of the filtrate A, a mass of the activated carbon is 5-8 g; and

(5) subjecting the filtrate C obtained in the step (4) to reduced-pressure distillation and cooling crystallization to obtain the bio-based succinic acid composition;

or,

the preparation method comprises the following steps:

(S) mixing and dissolving the bio-based succinic acid with the readily oxidizable substance content of $\geq$ 0.35 mL/g and a solvent at 60-100°C, then cooling to 15-25°C at a rate of 5-15°C/h for recrystallization, performing filtration, and repeating the aforementioned steps at least once to obtain the bio-based succinic acid composition.

8. A polyester, wherein raw materials for preparing the polyester comprise the bio-based succinic acid composition according to any one of claims 1-3.

9. The polyester according to claim 8, comprising the following components:

component A:
based on a total molar amount of 100 mol% of the component A, being a dicarboxylic acid compound comprising the following components:

a1, being 65-100 mol% of the bio-based succinic acid composition according to any one of claims 1-3 and/or a derivative of an ester thereof, and
a2, being 0-35 mol% of adipic acid and/or a derivative of an ester thereof;
and

component B: being 1,4-butanediol with a molar amount at least equal to that of the component A.

10. The polyester according to claim 8, wherein according to standard DIN EN 12634-1998, an acid number of the polyester is ≤ 1.20 mg KOH/g;

according to standard GB/T 14190-2008 and using a CIE 1976 (L*a*b*) color space, an a value of the polyester is ≤ 1.5; and
an intrinsic viscosity of the polyester determined according to GB/T 17931-1999 is ≥ 1.40 dL/g.

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5143833 A
- US 5504004 A
- US 5770435 A
- JP H011113588 B
- CN 103183813 A
- CN 118459331 A